# EUROPÄISCHE PATENTSCHRIFT

(11) **EP 0 665 218 B1**
(45) Veröffentlichungstag und Bekanntmachung des Hinweises auf die Patenterteilung: **24.03.1999**
(21) Anmeldenummer: 94903173.6
(22) Anmeldetag: 16.11.1993
(51) Int. Cl.: C07C 311/39, C07C 303/44

(54) **VERFAHREN ZUR HERSTELLUNG EINER PHYSIKALISCH STABILEN, KRISTALLINEN GAMMA-MODIFIKATION VON PARA-AMINOBENZOLSULFONAMID**
METHOD FOR THE PREPARATION OF PHYSICALLY STABLE CRYSTALLINE GAMMA-MODIFICATION OF PARA-AMINOBENZENESULFONAMIDE
PROCEDE DE PREPARATION D'UNE MODIFICATION GAMMA CRISTALLINE PHYSIQUEMENT STABLE DE PARA-AMINOBENZENESULFONAMIDE

(30) Priorität: 19.08.1993 RU 93041684
(43) Veröffentlichungstag der Anmeldung: 02.08.1995
(73) Patentinhaber: LEONIDOV, Nikolai Borisovich, Moskau, 115407 (RU)
(72) Erfinder: LEONIDOV, Nikolai Borisovich, Moskau, 115407 (RU)
(74) Vertreter: Beetz & Partner Patentanwälte
(86) Internationale Anmeldenummer: RU9300274
(87) Internationale Veröffentlichungsnummer: WO9505361

(56) Entgegenhaltungen:
- DE-C- 704 447
- GB-A- 513 242
- SU-A- 136 363
- SU-A- 433 672
- US-A- 5 602 281
- M.D.Mashkowsky: "Lekarstwennye sredstwa", Medizina 1993, Seite 321
- THE MERCK INDEX, 1989, Eintrag 798, Seite 1280
- English-Russian Dictionary of Chemistry and Chemical Technology, 1966, Publishing House Sovetskaya Encyclopedia, Seite 633

## Beschreibung

Die Erfindung bezieht sich auf den Bereich der organischen Chemie, insbesondere auf ein Verfahren zur Herstellung einer physikalisch stabilen kristallinen γ-Modifikation von para-Aminobenzolsulfonamid.

### Vorbekannter Technikstand

Bekannt ist, dass para-Aminobenzolsulfonamid kristallisierbar ist, und zwar in Form von mindestens drei polymorphen Modifikationen, bezeichnet als α-, β - und γ-Formen (Journal of Pharmaceutical Sciences, v. 59, No. 7, July 1970, p. 972-975; Journal of Pharmaceutical of Japan, 1942, v. 63, No. 11, p. 17-19), von denen lediglich die α-Form Anwendung in der Medizinpraxis findet. Diese polymorphen Modifikationen werden durch Rekristallisation und Umwandlungen der einen Form zu einer anderen hergestellt.

Die kristalline γ-Modifikationen von para-Aminobenzolsulfonamid stellt man durch Auflösung der β-Modifikation in Amylalkohol unter anschliessendem Sieden her. Die sichbildenden und ausscheidenden Kristalle werden in eine Wärmeisolierung eingebracht, allmählich abgekühlt (auf Raumtemperature) und dann filtriert. Durch Röntgenstrukturanalyse wird die γ-Modifikation identifiziert.

Vorbekannt ist ferner ein Verfahren zur Herstellung der γ-Modifikation von para-Aminobenzolsulfonamid durch Pulverisierung der α- und β-Modifikationen und anschliessende Aufwärmung des hergestellten Pulvers 1 Stunde lang bei einer Temperatur 130÷140°C. Dabei erfolgt ein Übergang der β-Modifikation in die γ-Modifikation. Die auf solchem Wege hergestellte γ-Modifikation von Aminobenzolsulfonamid ist jedoch unstabil bei Raumtemperatur und verwandelt sich in die β-Modifikation, welche im weiteren spontan in die die α-Modifikation übergeht. Infolge der Unstabilität der hergestellten gamma-Modifikation bleibt ihre pharmakologische Wirkung noch nicht erforscht. Die obigen Verfahren zur Herstellung der gamma-Modifikation von para-Aminobenzolsulfonamid konnten keine industrielle Realisierung finden.

### Offenbarung der Erfindung

Der vorliegenden Erfindung liegt die Aufgabe zugrunde, durch Änderung technologischer Verfahrensschritte ein Verfahren zu entwickeln, welches es ermöglicht, eine physikalisch stabile kristalline gamma-Modifikation von para-Aminobenzolsulfonamid herzustellen, die eine hohe Antimikroben- und interferoninduzierende Wirkung aufweist.

Die Aufgabe ist dadurch gelöst, dass bei dem angemeldeten Verfahren zur Herstellung einer physikalisch stabilen kristallinen gamma-Modifikation von para-Aminobenzolsulfonamid erfindungsgemäss eine Lösung von para-Aminobenzolsulfonamid in Wasser oder organischem Lösungsmittel oder in deren Mischung mittels eines Kältemittels mit einer Geschwindigkeit von mindestens 2°C/Minute auf deren vollständige Kristallisation abgekühlt wird und man anschliessend die erzeugten Kristalle ausscheidet und trocknet.

Als organisches Lösungsmittel sind zweckmässigerweise niedere Alkohole, vorzugsweise Äthanol, zu verwenden. Zwecks erhöhter Ausbeuten des Endproduktes ist es zweckmässig, flüssigen Stickstoff oder flüssiges Kohlenstoffdioxid als Kältemittel einzustzen und die Trocknung durch Vakuumierung bei einem Druck von höchstens 1.33 Pascals (10⁻² mm QS-Säule) vorzunehmen. Das angemeldete Verfahren ermöglicht es, eine physikalisch stabile kristalline gamma-Modifikation von para-Aminobenzolsulfonamid herzustellen, welche lagerungsbeständing bei Raumtemperatur ist und eine Antimikrobenwirkung aufweist, die die der β- und α-Formen in der Medizinpraxis übersteigt. Darüber hinaus besitzt die gamma-Modifikation eine hocheffektive interferoninduzierende Wirkung. Das Verfahren kennzeichnet sich durch technologische Einfachheit und industrielle Realisierbarkeit.

### Beste Durchführungsvariante der Erfindung

Das angemeldete Verfahren zur Herstellung einer physikalisch stabilen kristallinen gamma-Modifikation von para-Aminobenzolsulfonamid wird durch Abkühlung einer Lösung von para-Aminobenzolsulfonamid in Wasser oder organischem Lösungsmittel oder in deren Mischung mittels eines Kältemittels bei einer Geschwindigkeit von mindestens 2°C/Minute bis zu deren vollständiger Kristallisation mit anschliessender Ausscheidung der entstehenden Kristalle und deren Trocknung verwirklicht. Als Kältemittel ist eine beliebige Substanz verwendbar, die die Temperatur des abzukühlenden Stoffes mit einer Geschwindigkeit von mindestens 2°C/Minute vermindern kann. Als optimales Kältemittel kommen flüssiger Stickstoff oder flüssiges Kohlendioxid in Frage, deren Einsatz es ermöglicht, die Ausbeute des Endproduktes auf dem Wege einer schnellen Erreichung und weiteren Aufrechterhaltung der erforderlichen Abkühlungsgeschwindigkeit zu erhöhen.

Die Herstellung der physikalisch stabilen kristallinen gamma-Modifikation der obigen Verbindung is bei der Abkühlungsgeschwindigkeit von mindestens 2°C/Minute erzielbar, eine Abkühlungsgeschwindigkeit kleiner als 2°C/Minute ermöglicht es nicht, eine neue kristalline Modifikation herzustellen. Der obere Grenzwert der Abkühlgeschwinäigkeit ist unbegrenzt: Bei beliebeger maximal erzielbarer Abkühlungsgeschwindigkeit der Ausgangslösung erfolgt die Bildung der neuen kristallinen Modifikation. Der Abkühlvorgang erfolgt in Wasser oder in beliebigem organischen Lösungsmittel oder in deren Mischung, in denen die Ausgangssubstanz löslich ist. Als vorzugsweise Lösungsmittel kommen Wasser und niedere Alkohole in Frage, von denen Äthanol zu bevorzugen ist. Dabei lässt sich eine höchste Ausbeute des Endproduktes erzielen. Die angemeldete Verbindung kann man unabhängig von der Konzentration der Ausgangssubstanz in der Lösung herstellen. Die Wahl der Trocknungsbetriebsweise bei einem Druck nicht höher als 1.33 Pascals (10⁻² mm QS-Säule) ist dadurch bedingt, dass das getrocknete Fertigprodukt eine Feuchtigkeit nicht grösser als 3% aufweisen soll. Das hergestellte Zielprodukt, nämlich die physikalisch stabile kristalline gamma-Modifikation von para-Aminobenzolsulfonamid, ist ein feinkristallines weisses Pulver (für die gamma> Modifikation typische Kristalle von eckiger Form). Eine Spektralanalyse und Röntgengramme bestätigen, dass das hergestellte Endprodukt die gamma-Modifikation der obengenannten Verbindung ist und sich durch folgende Reihe der Werte der Interebeneabstände d und der relativen Reflexintensitäten I kennzeichnet :

| d, A | I |
|---|---|
| 7,63 | 31 |
| 6,61 | 73 |
| 6,28 | 8 |
| 6,00 | 3 |
| 5,64 | 30 |
| 4,90 | 71 |
| 4,51 | 100 |
| 4,27 | 4 |
| 4,19 | 5 |
| 3,80 | 98 |
| 3,66 | 30 |
| 3,56 | 32 |
| 3,47 | 4 |
| 3,36 | 4 |
| 3,23 | 8 |
| 3,20 | 12 |
| 3,13 | 13 |
| 3,04 | 30 |
| 2,99 | 25 |
| 2,81 | 13 |
| 2,67 | 2 |
| 2,64 | 3 |
| 2,62 | 2 |
| 2,53 | 5 |
| 2,49 | 10 |
| 2,46 | 6 |
| 2,33 | 9 |
| 2,25 | 6 |
| 2,22 | 4 |
| 2,21 | 4 |
| 2,17 | 4 |
| 2,16 | 4 |
| 2,14 | 4 |
| 2,12 | 8 |
| 2,11 | 9 |
| 1,99 | 5 |
| 1,97 | 6 |
| 1,93 | 4 |
| 1,92 | 5 |
| 1,91 | 5 |
| 1,88 | 5 |
| 1,86 | 4 |
| 1,84 | 3 |
| 1,82 | 4 |
| 1,80 | 5 |
| 1,77 | 3 |
| 1,75 | 3 |
| 1,727 | 5 |
| 1,618 | 4 |
| 1,583 | 3 |
| 1,566 | 4 |

Die hergestellte physikalisch stabile kristalline gamma-Modifikation von para-Aminobenzolsulfonamid weist eine Antimikroben- und Interferoninduzierwirkung auf.

Die Aktivität der hergestellten gamma-Modifikation wurde in einem Experiment an Tieren im Vergleich zu dem in der Medizinpraxis verwendeten offizinellen para-Aminobenzolsulfonamid, das hauptsächlich die α-Modifikation enthält, untersucht.

Zur Untersuchung der spezifischen Antimikrobenwirkung verwendete man die Methode von Serienaufzuchtexperimenten. Als Ausgangslösungen dienten 0,5%ige Lösungen von einem offizinellen para-Aminobenzolsulfonamid und der gamma-Modifikation dieser Verbindung in einer 0,01-n-Lösung von Natriumhydroxid. Zweifache Aufzuchtfolgen der zu prüfenden Lösungen in einer Fleisch-Pepton-Brühe mit Zugabe einer Kultur von Staphylococcus aureus wurden vorbereitet. Die Aussaat wurde 24 Stunden lang in einem Thermostat gehalten. Die bakteriostatische Konzentration wurde visuell eingeschätzt.

Man verwendete sterile und unsterile Lösungen von para-Aminobenzolsulfonamid und dessen gamma-Modifikation. Die Experimente wurden an drei Serien der zu untersuchenden Lösungen mit dreimaliger Wiederholung durchgeführt. Die Ergebnisse sind in Tabelle 1 dargestellt. Eine Analyse der Tabelle 1 ergibt, dass die gamma-Modifikation eine Antimikrobenwirkung aufweist, die die von dem offizinellen para-Aminobenzolsulfonamid übertrifft.

**Tabelle 1**

| Nr. | Zu untersuchende Präparate | Zeit, Tage Wachstum (visuell) | | |
|---|---|---|---|---|
| | | 0 | 1 | 2 |
| 1 | 2 | 3 | 4 | 5 |
| 1 | steriles Lösungsmittel | kein Wachstum | Wachstum | Wachstum |
| 2 | para-Aminobenzolsulfonamid | kein Wachstum | kein Wachstum | kein Wachstum |
| 3 | gamma-Modifikation von para Aminobenzolsulfonamid | kein Wachstum | kein Wachstum | kein Wachstum |
| 4 | unsteriles Lösungsmittel | kein Wachstum | Wachstum | Wachstum |
| 5 | para-Aminobenzolsulfonamid (pharmakop.), unsteril | kein Wachstum | kein Wachstum | kein Wachstum |
| 6 | gamma-Modifikation von para-Aminobenzolsulfonamid, unsteril | kein Wachstum | kein Wachstum | kein Wachstum |

| | | 3 | Kontrolle | |
|---|---|---|---|---|
| 1 | 2 | 6 | 7 | |
| 1 | steriles Lösungsmittel | Wachstum | Wachstum | |
| | | | | |
| 2 | para-Aminobenzolsulfonamid | Wachstum | Wachstum | |
| | | | | |
| 3 | gamma-Modifikation von para-Aminobenzolsulfonamid | Wachstum | Wachstum | |
| | | | | |
| 4 | unsteriles Lösungsmittel | Wachstum | Wachstum | |
| | | | | |
| 5 | para-Aminobenzolsulfonamid (pharmakop.), unsteril | Wachstum | Wachstum | |
| | | | | |
| 6 | gamma-Modifikation von para-Aminobenzolsulfanylamid, unsteril | Wachstum | Wachstum | |

Wie die Untersuchung der Toxizitätseffekte bei der gamma-Modifikation von para-Aminobenzolsulfonamid im Vergleich zu dem offizinellen para-Aminobenzolsulfonamid ergeben konnte, weist die gamma-Modifikation eine kleinere Toxizität auf, und zwar beträgt LD₅₀ bei intraabdominaler Injizierung Ratten 1918 mg/kg Körpergewicht der Tiere, während LD₅₀ des offizinellen Präparates 1242 mg/kg Körpergewicht beträgt. Die interferoninduzierende Wirkung der gamma-Modifikation von para-Aminobenzolsulfonamid im Vergleich zu dem offizinellen para-Aminobenzolsulfonamid wurde an Versuchsmäusen in vivo untersucht.

Bei solchen Experimenten kamen Mausmännchen der Linie CBA mit 10-12 g Körpergewicht in Frage. Als Zellenkultur verwendete man eine durchflechtbare Zellenlinie von Mäusefibroblasten Z-929. Die Zellen wurden in 96-Pflanzlöcherplatten aus Kunststoff (37°C, 3,5% CO), im Eagle-Medium 2 MEM 10% des Rindviehserums aufgezüchtet. Als Virus wählte man den Enzephalocarditisvirus der Mäuse, Stamm Columbia.

Das offizinelle Präparat und die gamma-Modifikation von para-Aminobenzolsulfonamid wurden in Dosen 50, 150 µg/0,2 ml, einmalig, intra-abdominal (0,2 ml je 1 Maus) injiziert.Die Blutentnahme erfolgte aus der Kopfschlagader, 5, 24 und 72 Stunden nach der Injizierung der Präparate. Für jede Probe dienten mindestens je 5 Tiere. Die Interferontitration wurde durch Ermittlung der Niederdrückung der zytopathischen Wirkung an der Zellenkultur mittels der Mikromethode vorgenommen. Die Untersuchungsergebnisse sind in Tabelle 2 dargestellt.

**Tabelle 2**

| Dynamik der Interferonbildung im Serum des Mäuseblutes bei Injizierung der zu testenden Präparate | | | |
|---|---|---|---|
| Nr. | Präparate | Zeit der Blutentnahme nach Präparatinjizierung, in Stunden | |
| 1 | 2 | 3 | |
| 1 | offizinelles para-Aminobenzolsulfonamid | 5 24 72 | |
| 2 | gamma-Modifikation von para-Aminobenzolsulfonamid | 5 24 72 | |
| 3 | Kontrolle | 5 24 72 | |
| 4 | Maus-Seruminterferon | - | |

| Nr. | Präparate | Interferontiter, Einheit pro ml | |
|---|---|---|---|
| | | Präparatkonzentration 50 µg je 1 1 Tier | Präparatkonzentration 150 µg je 1 Tier |
| 1 | 2 | 4 | 5 |
| 1 | offizinelles para-Aminobenzolsulfonamid | unter 20 | unter 20 |
| | | unter 20 | unter 20 |
| | | unter 20 | unter 20 |
| 2 | gamma-Modifikation von para-Aminobenzolsulfonamid Kontrolle | 40-80 | 80 |
| | | 160 | 320 |
| | | unter 20 | unter 20 |
| 3 | Kontrolle | unter 20 | |
| | | unter 20 | |
| | | unter 20 | |
| 4 | Maus-Seruminterferon | 640 | |

Eine Analyse der Untersuchungsergebnisse zeigte, dass das offizinelle para-Aminobenzolsulfonamid keine interferoninduzierende Wirkung aufweist. Die gamma-Modifikation dieser Verbindung induzierte dagegen ein Interferon im Blutserum der Mäuse bereits nach 5 Stunden nach der Injizierung (Frühinterferon) mit einer Aktivität 40-80 Einheiten pro ml, und nach 24 Stunden erreichten die Interferontiter bereits 160-320 Einheiten/ml. Gegen 72 Stunden nach der Injizierung erfolgte eine Verminderung der Interferontiter. Die kristalline gamma-Modifikation von para-Aminobenzolsulfonamid, hergestellt durch das angemeldete Verfahren, ist also eine physikalisch stabile Form mit einer hocheffektiven Antimikroben- und interferoninduzierenden Aktivität.

Zur besseren Erfassung der vorliegenden Erfindung dienen die nachstehenden Beispiele für die Herstellung der obenbeschriebenen physikalisch stabilen kristallinen gamma-Modifikation von para-Aminobenzolsulfonamid.

### Beispiel 1.

Man kühlt 1,5 l einer wässrigen Lösung von para-Aminobenzolsulfonamid (20 g/l) mit flüssigem Stickstoff bei einer Geschwindigkeit 2°C/Minute bis zu deren vollständigen Kristallisation ab. Die erhaltene eingefrorene Masse wird auf Untersätze gelegt und in einen Sublimator eingebracht. Die Trocknung erfolgt bei einem Druck 1.33 Pascals (10⁻² mm QS) bis zu einer Restfeuchtigkeit 3%. Die Endproduktausbeute beträgt 30 g (100%). Das hergestellte Produkt is ein feinkristallines weises Pulver und wird anhand von Werten der Interbenenabstände d und von relativen Reflexen I charakterisiert, welche mit den entsprechenden obenangegebenen Werten der kristallinen gamma-Modifikation von para-Aminobenzolsulfonamid zusammenfallen.

### Beispiel 2.

Der Prozess wird ähnlich dem in Beispiel 1 beschriebenen durchgeführt, dabei verwendet man 500 ml Lösung des Ausgangsstoffes im Gemisch Wasser/Äthanol (1:1) mit einer Konzentration 20 g/l. Die Endproduktausbeute beträgt 96 Gew.-%. Die hergestellte Substanz weist die dem Beispiel 1 ähnlichen Kennwerte auf.

### Beispiel 3.

Der Prozess wird ähnlich dem in Beispiel beschriebenen mit einer Kühlgeschwindigkeit 30°C/Minute durchgeführt. Die Endproduktausbeute beträgt 98,2 Gew.-%. Die hergestellte Substanz weist die dem Beispiel 1 ähnlichen Kennwerte auf.

### Beispiel 4.

1,5 l einer Lösung von para-Aminobenzolsulfonamid in Äthanol (10 g/l) kühlt man mit flüssigem Stickstoff mit einer Geschwindigkeit 8°C/Minute bis zu deren vollständigen Kristallisation ab. Als Kältemittel dient flüssiges CO₂. Die hergestellte eingefrorene Masse wird anschliessend in einen Sublimator eingebracht und bei einem Druck 1.33 Pascals (10⁻² mm QS) getrocknet. Die Endproduktausbeute beträgt 95,6 Gew.-%. Die hergestellte Verbindung weist die dem Beispiel 1 ähnlichen Kennwerte auf.

### Beispiel 5.

Der Prozess wird ähnlich wie im Beispiel 1 durchgeführt, die Konzentration der Ausgangsverbindung in Äthanol beträgt 10 g/l, als Kältemittel dient flüssiges CO₂. Die Endproduktausbeute beträgt 96,8 Gew.-%. Die hergestellte Substanz weist die dem Beispiel ähnlichen Kennwerte auf.

### Industrielle Anwendbarkeit

Die gemäss dem angemldeten Verfahren hergestellte physikalisch stabile kristalline gamma-Modifikation weist hocheffektive Antimikroben- und interferoninduzierende Wirkung und findet in der Medizinpraxis als. Wirkstoff eines Arzneimittels Verwendung.

## Patentansprüche

1. Verfahren zur Herstellung einer physikalisch stabilen kristallinen gamma-Modifikation von para-Aminobenzolsulfonamid, **dadurch gekennzeichnet,** dass man eine Lösung von para-Aminobenzolsulfonamid in Wasser oder einem organischen Lösungsmittel, oder in deren Gemisch mittels eines Kältemittels mit einer Geschwindigkeit von mindestens 2°C/Minute bis zu deren vollständiger Kristallisation unter anschliessender Ausscheidung der sich bildenden Kristalle und deren Trocknung abkühlt.

2. Verfahren nach Anspruch 1, **dadurch gekennzeichnet,** dass als organisches Lösungsmittel niedere Alkohole verwendet werden.

3. Verfahren nach Anspruch 2, **dadurch gekennzeichnet,** dass als niederer Alkohol Äthanol verwendet wird.

4. Verfahren nach beliebigem der Ansprüche 1-3, **dadurch gekennzeichnet,** dass man als Kältemittel flüssigen Stickstoff oder flüssiges Kohlenstoffdioxid verwendet und die Trocknung durch Vakuumierung bei einem Druck von mindestens 1.33 Pascals (10⁻² mm Quecksilbersäule) vornimmt.

## Claims

1. A method of producing a physically stable crystalline γ-modification of paraaminobenzenesulphonamide, characterised in that a solution of para-aminobenzenesulphonamide in water or in an organic solvent or in a mixture thereof is chilled by means of a refrigerant at a rate of at least 2°C per minute until the said solution has crystallised completely, the crystals forming being subsequently separated out and dried.

2. A method as claimed in claim 1, characterised in that lower alcohols are used as organic solvent.

3. A method as claimed in claim 2, characterised in that the lower alcohol used is ethanol.

4. A method as claimed in any one of claims 1 to 3, characterised in that the refrigerant used is liquid nitrogen or liquid carbon dioxide, and in that drying is carried out by vacuum drying at a pressure of at least 1.33 Pa (10⁻² mm Hg).

## Revendications

1. Procédé de préparation d'une forme cristalline - gamma physiquement stable d'une para-aminobenzène sulfonamide caractérisé en ce qu'on refroidit, à une vitesse d'au moins 2°C/minute, une solution de para-aminobenzène sulfonamide, dans l'eau ou dans un solvant organique ou leur mélange à l'aide de moyens de refroidissement, jusqu'à sa cristallisation complète avec finalement séparation des cristaux formés et leur séchage.

2. Un procédé selon la revendication 1, caractérisé en ce que, en tant que solvant organique, on utilise des alcools inférieurs.

3. Un procédé selon la revendication 2, caractérisé en ce que, en tant qu'alcool inférieur, on utilise l'éthanol.

4. Un procédé selon une quelconque des revendications 1 à 3, caractérisé en ce que en tant que moyens de refroidissement, on utilise de l'azote liquide ou du dioxyde de carbone liquide et qu'on effectue le séchage par application d'un vide d'une pression d'au moins 1,33 Pascals (10⁻² mm Hg).
